# EUROPEAN PATENT SPECIFICATION

(11) **EP 3 865 174 B1**
(45) Date of publication and mention of the grant of the patent: **28.10.2026**
(21) Application number: 21167645.7
(22) Date of filing: 20.02.2014
(51) Int. Cl.: A61M 39/26

(54) **NEEDLELESS CONNECTOR WITH FOLDING VALVE**
NADELLOSER VERBINDER MIT FALTBAREM VENTIL
CONNECTEUR SANS AIGUILLE AVEC VALVE PLIABLE

(30) Priority: 13.03.2013 US 201313801412
(43) Date of publication of application: 18.08.2021
(62) Document number(s) of the earlier application(s) in accordance with Art. 76 EPC: 14709077.3 / 2 968 906
(73) Proprietor: CareFusion 303, Inc., San Diego, CA 92130 (US)
(72) Inventor: YEH, Jonathan, San Diego, CA 92130 (US); ZOLLINGER, Christopher J., Chino Hills, CA 91709 (US); QUACH, Matthew, San Gabriel, CA 91775 (US); MANSOUR, George Michel, San Diego, CA 92130 (US)
(74) Representative: Epping - Hermann - Fischer

(56) References cited:
- WO-A1-2011/014265
- WO-A1-2011/060384
- WO-A2-2008/091698
- WO-A2-2013/016077
- US-A1- 2011 130 724

## Description

### Cross-references to related applications

Not applicable.

### BACKGROUND

### Field

The present disclosure generally relates to needleless connectors, and, in particular, to connectors with an internal collapsible valve.

### Description of the Related Art

Medical treatments often include the infusion of a medical fluid, for example a saline solution or a liquid medication, to patients using an intravenous (IV) catheter that is connected though an arrangement of flexible tubing and fittings, commonly referred to as an "IV set," to a source of fluid, for example an IV bag. The fittings commonly include interconnectable male and female needleless connectors having a "Luer taper" conforming to an International Standards Organization (ISO) standard. Certain connectors have a self-sealing feature to prevent leakage of fluid from the attached tubing when the connector is decoupled from a mating connector.

US 2011/130724 A1 discloses a connector that has a housing having a fluid path from a first port through an internal cavity to a second port. The connector also includes a plug that has first and second positions within the internal cavity, where the plug blocks the fluid path between the first port and the internal cavity when in the first position. The plug has a diaphragm that separates the internal cavity into a first volume that is vented and a second volume that includes the fluid path. The plug also includes a biasing element that is disposed within the first volume and that urges the plug toward the first position. Displacement of the plug from the first position toward the second position opens the fluid path and increases the second volume.
WO 2013/016077 A2 discloses a connector that includes a single fluid flow path, an air chamber and a valve. The single fluid flow path is configured for delivering fluid to a person and configured for receiving fluid from a person. The air chamber configured for expelling air from the air chamber when an actuator is inserted into the connector and for receiving air into the chamber when the actuator is removed from the connector. The valve plug comprises two diaphragms that separate the air chamber from the single fluid flow path. The valve plug is configured for creating positive displacement by returning the valve plug to its uncompressed state when the actuator is removed from the connector.
WO 2011/014265 A1 discloses a collapsible valve comprising a first portion with at least one dimple in a side thereof, and a second portion, the second portion being narrower than the first portion and arranged along an axial dimension of the first portion, the second portion including a cut therein.
WO 2011/060384 A1 discloses a collapsible valve for use in a needleless access connector to reduce the priming volume of the needleless access connector.

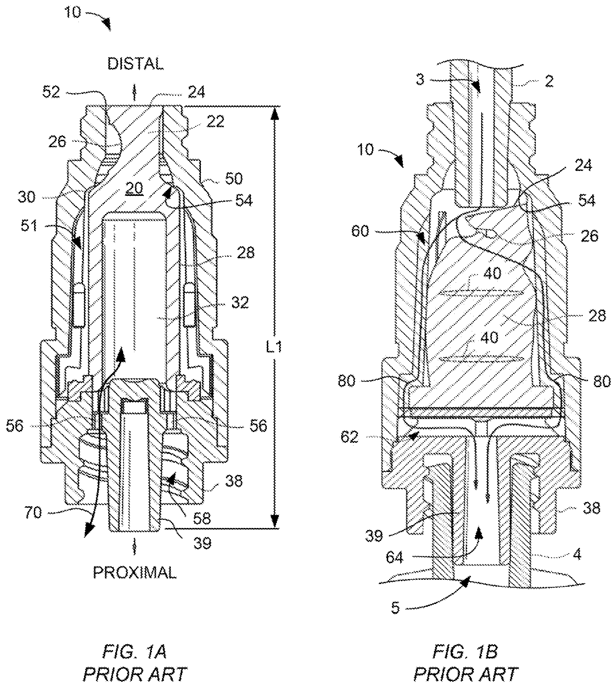

One conventional needleless valve, shown in FIGS. 1A-1B, has a collapsible internal valve made of a flexible material. When a force is applied to the top of the valve by the tip of a male Luer connector, the valve folds at a "smiley cut" located in the upper portion, referred to as the "head" of the valve, thereby opening a flow path through the connector. As the size of this type of connector is reduced, however, the behavior of the flexible valve may not scale and the valve having a single smiley cut may not fold at the desired amount of force.

Most needleless connectors trap some amount of fluid when the connector is disconnected from a previously mated connector. As some medical fluids degrade with time, this trapped fluid may present a hazard to a patient.

### SUMMARY

The invention is defined by the claims. In the following, parts of the description and drawings referring to embodiments which are not covered by the claims are not presented as embodiments of the invention, but as examples useful for understanding the invention.
The self-sealing needleless female Luer connector disclosed herein is reduced in size compared to conventional connectors of the same type and, therefore, may trap a reduced amount of fluid within the connector upon disconnection. The disclosed connector also accepts a standard male Luer fitting and provides a self-sealing port with a continuous external surface at the port when the connector is not activated such that the port may be disinfected prior to use.

In certain embodiments, a needleless connector is disclosed that includes a body having an internal cavity with a sealing ridge, a port, an output flow channel, and a fluid flow path between the port and output flow channel. The connector also includes a collapsible valve disposed within the cavity. The valve has a cylindrical wall having a center axis and a shoulder and defining an internal air space, wherein the shoulder is configured to sealingly contact the ridge of the body so as to block the fluid flow path, and a head fixedly attached to the wall. The head has first and second smiley cuts disposed on opposite sides of the head.

In certain embodiments, a collapsible valve is disclosed that includes a cylindrical wall having a center axis and defining an internal air space and a head fixedly attached to the wall. The head has first and second smiley cuts disposed on opposite sides of the head.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
FIGS. 1A-1B are cross-sections of a conventional needleless connector.
FIGS. 2A-2B are cross-sections of an exemplary needleless connector according to certain aspects of the present disclosure.
FIGS. 3-5 are side views of various example embodiments of the head of a valve according to certain aspects of the present disclosure.

### DETAILED DESCRIPTION

It is advantageous to provide a self-sealing, needleless connector that accepts male Luer fittings that meet the ISO standard while the size of the connector, and therefore the volume of fluid within the connector, is reduced compared to conventional connectors.

In the following detailed description, numerous specific details are set forth to provide a full understanding of the present disclosure. It will be apparent, however, to one ordinarily skilled in the art that embodiments of the present disclosure may be practiced without some of the specific details. In other instances, well-known structures and techniques have not been shown in detail so as not to obscure the disclosure. In the referenced drawings, like numbered elements are the same or essentially similar. Reference numbers may have letter suffixes appended to indicate separate instances of a common element while being referred to generically by the same number without a suffix letter.

FIGS. 1A-1B are cross-sections of a conventional needleless connector 10. With reference to FIG. 1A, the connector 10 includes a collapsible valve 20 disposed within a cavity 51 of body 50. The valve 20 has a shoulder 30 that continuously contacts a ridge 54 within the cavity 51 when the connector 10 is de-activated, *i.e.* not connected to a mating connector, to form a primary seal that blocks the fluid flow path through the connector 10. The valve 20 has an internal air space 32 that is separated from the cavity 51 by a cylindrical wall 28. The air space 32 is vented to the ambient environment through air passages 56 and the external cavity 58 within the threaded connector 38 surrounding the male Luer fitting 39 of the body 50, as indicated by the air flow path 70. The valve 20 also has a solid head 22 with a "smiley cut" 26 formed on one side and a top surface 24 that is positioned generally flush with a port 52 of the cavity 51 when the connector 10 is de-activated. The edge of the top surface 24 seals to the port 52. The top surface 24 is continuous, *i.e.* there is no slit or penetration in the surface that may trap bacteria or other contamination. The conventional connector 10 has a representative overall length of L1. In certain conventional valves, L1 = 3.363 cm (1.324 inches).

FIG. 1B depicts the valve 10 in the activated position, *i.e.* a male Luer fitting 2 sealingly coupled to the connector 10 at port 52. The tip of the male Luer fitting 2 has displaced the external surface 24 downward and the applied force has caused the head 22 to buckle toward the smiley cut 26 as well as causing the cylindrical wall 28 to buckle. Two external dimples 40 (indicated in phantom) are located on an exterior surface (not visible in the cross-section of FIG. 1B) of the wall 28. The dimples 40 control the collapse of the wall 28 to occur at a predictable level of force. In the activated configuration, the primary seal between the shoulder 30 and ridge 54 is opened such that fluid may flow through the connector 10, as indicated by the fluid flow path 80 that passes from the lumen 3 of the male Luer fitting 2 through the cavity 51 and through channels 62 in the base and out through an output flow channel 64 of the male fitting 39 that is fluidly coupled to the flow channel 5 of the connected female Luer fitting 4. Air passes out of the air chamber 32 along the air flow path 70 as the valve 20 collapses.

While the conventional needleless connector 10 of FIGS. 1A, 1B allows the connection and disconnection of a male Luer fitting and seals the flow path when there is no fitting mated with the connector 10, it is generally desirable to have the least possible amount of fluid contained in the needleless connector. To this end, the disclosed needleless connector 100 provides the same functionality of allowing the connection and disconnection of the same male Luer fitting and sealing of the flow path when there is no fitting mated, with the improvement of a smaller body that results in a smaller internal volume of fluid compared to the conventional connector 10. As a medication that remains trapped in the connector may not reach a patient, unless the connector is flushed with a medical liquid such as a saline solution, reducing the liquid volume of a needleless connector increases the amount of the medication that reaches the patient. In addition, as medications may degrade over time and connectors may not always be flushed after administration of the medication through a needleless connector, a reduction in the trapped volume of a medication in a connector necessarily reduces the amount of degraded medication that may reach the patient at a later time.

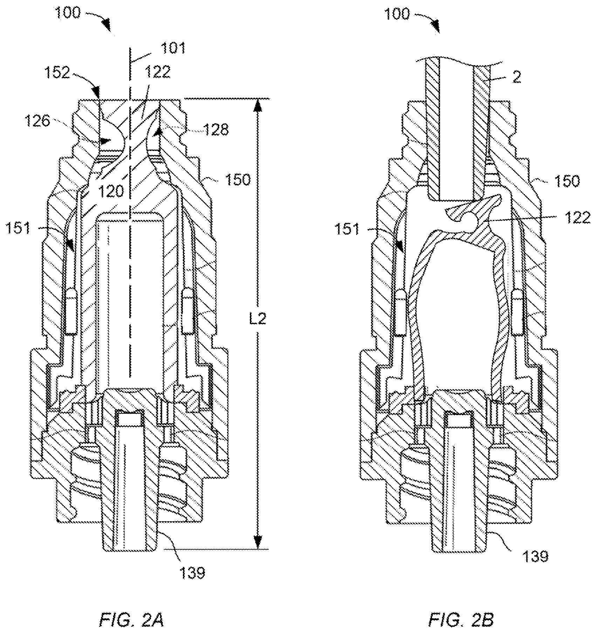

FIGS. 2A-2B are cross-sections of an exemplary needleless connector 100 according to certain aspects of the present disclosure. The connector 100 has a body 150 that defines a cavity 151. The connector 100 has a characteristic length L2, which references the same features as the characteristic length L1, this is less than L1. In certain embodiments, L2 is less than 90% of L1. In certain embodiments, L2 = 3.048 cm (1.200 inches). The smaller body 150 contains less fluid than the body 50 of the conventional connector 10. The body 150 of the connector 100 is generally similar to the body 150 of connector, although the port 152 and the male Luer fitting 139 meet the same ISO standards as port 52 and fitting 39 of connector 10. In certain embodiments, the male Luer fitting 139 may be replaced with a tubing connector (not shown) that accepts an end of a length of tubing, for example when a connector 100 is integrated into an IV set.

As the size of the connector 100 is reduced compared to connector 10, the volume of the cavity 151 that is external to the valve 120 is also reduced compared to connector 10. As the wall thickness of the body 150 and some internal features must remain the same as body 50, for example to provide a minimum thickness for flow of molten plastic in a molding process, the reduction in the fluid volume may be proportionately greater than the reduction in a linear dimension. In certain embodiments, the reduction in fluid volume may be 40% while the reduction in the linear dimension L2 vs. L1 may be only 10%.

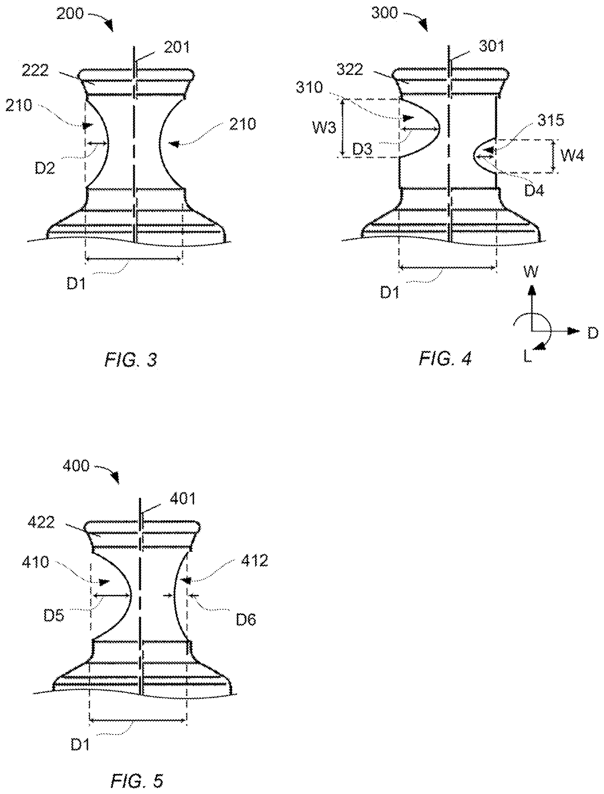

The connector 100 has a valve 120 disposed within the cavity 151 that is generally similar to the valve 20 of connector 10. The valve 120 comprises a flexible material, for example silicone. Valve 120 has a center axis 101 and a head 122 with two smiley cuts 126, 128 disposed on opposite sides of the head. The details of the smiley cuts are discussed in greater detail in FIGS. 3-5.

FIGS. 3-5 are cross-sections of various example embodiments of a valve according to certain aspects of the present disclosure. FIG. 3 depicts the head 222 of a valve 200 that is similar to valve 120 of FIG. 2. The valve 200 has two identical smiley cuts 210 that are equally spaced from a center axis 201 of the valve 200 and have a common depth D2. The smiley cuts 210 have a uniform profile, *i.e.* the shape is the same over the length of the smiley cut, through the head 222. In FIG. 3, the directions of length, width, and depth of the smiley cuts are shown by the coordinate legend in FIG. 3, wherein the curved arrow indicates that length "L" is defined as directed into the plane of the drawing. The length of a smiley cut is defined as the value of the uniform profile in the direction of length L as measured at the maximum depth D. In certain embodiments, the depth D2 is defined at the center of the profile. In certain embodiments, the profile is symmetric. In certain embodiments, the profile is not symmetric. In certain embodiments, the profile has a constant radius. In certain embodiments, the profile has a variable radius. In certain embodiments, D2 is less than or equal to 30% of a diameter D1 of the head 222. In certain embodiments, D2 is less than or equal to 25% of D1. In certain embodiments, D1 = 0.381 cm (0.150 inches). In certain embodiments, D2 = 0.096 cm (0.038 inches).

FIG. 4 depicts the head 322 of a valve 300 that is similar to valve 120 of FIG. 2. The valve 300 has smiley cuts 310, 315 that are disposed on opposite sides of head 322 with depths D3, D4 respectively. In certain embodiments, D3 is equal to or greater than 30% of D1 while D4 is less than or equal to 25% of D1. In this example, smiley cut 315 is axially offset along the center axis 301 from smiley cut 310. In this example, the smiley cuts 310, 315 each have a parabolic profile, wherein the profile of smiley cut 310 is not the same profile as smiley cut 315. In certain embodiments, D3 and D4 are equal while smiley cuts 310, 315 have different parabolic profiles such that the respective widths W3, W4 of the smiley cuts 310, 315 are not equal. In certain embodiments W3 and W4 are equal.

FIG. 5 depicts the head 422 of a valve 400 that is similar to valve 120 of FIG. 2. The valve 400 has smiley cuts 410, 412 that are disposed on opposite sides of head 422. In this example, smiley cuts 410, 412 are axially aligned with different depths D5, D6. In certain embodiments, D5 is equal to or greater than 50% In certain embodiments, D6 is less than or equal to 20% In this example, smiley cut 410 has a parabolic profile while smiley cut 412 has a constant radius profile.

It can be seen that the disclosed embodiments of the needleless connector have a reduced internal volume of fluid while providing a self-sealing connection port of the same size and configuration of a conventional needleless connector. This reduction in fluid volume reduces the amount of fluid that remains trapped in the disclosed needleless connector when a connector attached to an IV line or a container such as a syringe is disconnected from the connector. While some amount of fluid is unavoidably retained in any needleless connector, reducing the amount of fluid that remains trapped in a connector increases the amount of an administered medication that reaches the patient. In addition, as medications may degrade over time, a reduction in the trapped volume of a medication in a connector necessarily reduces the amount of degraded medication that may reach the patient at a later time.

The previous description is provided to enable any person skilled in the art to practice the various aspects described herein. While the foregoing has described what are considered to be the best mode and/or other examples, it is understood that various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the terms "a set" and "some" refer to one or more. Pronouns in the masculine (e.g., his) include the feminine and neuter gender (e.g., her and its) and vice versa. Headings and subheadings, if any, are used for convenience only and do not limit the invention.

It is understood that the specific order or hierarchy of steps in the processes disclosed is an illustration of exemplary approaches. Based upon design preferences, it is understood that the specific order or hierarchy of steps in the processes may be rearranged. Some of the steps may be performed simultaneously.

Terms such as "top," "bottom," "front," "rear" and the like as used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

A phrase such as an "aspect" does not imply that such aspect is essential to the subject technology or that such aspect applies to all configurations of the subject technology. A disclosure relating to an aspect may apply to all configurations, or one or more configurations. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. A phrase such an embodiment may refer to one or more embodiments and vice versa.

The word "exemplary" is used herein to mean "serving as an example or illustration." Any aspect or design described herein as "exemplary" is not necessarily to be construed as preferred or advantageous over other aspects or designs.

All structural and functional equivalents to the elements of the various aspects described throughout this disclosure that are known or later come to be known to those of ordinary skill in the art are expressly incorporated herein by reference and are intended to be encompassed by the disclosure Moreover, nothing disclosed herein is intended to be dedicated to the public regardless of whether such disclosure is explicitly recited in the claims. Furthermore, to the extent that the term "include," "have," or the like is used in the description or the claims, such term is intended to be inclusive in a manner similar to the term "comprise" as "comprise" is interpreted when employed as a transitional word in a claim.

## Claims

1. A needleless connector comprising:
a body (150) having an internal cavity (151), a port (152), an output flow channel, and a fluid flow path between the port (152) and output flow channel; and
a collapsible valve (200, 300, 400) disposed within the internal cavity (151), the collapsible valve (200, 300, 400) comprising:
a cylindrical wall having a center axis (201, 301, 401) and a shoulder and defining an internal air space, wherein the shoulder is configured to sealingly contact a ridge of the body (150) so as to block the fluid flow path; and
a head (322, 422) fixedly attached to the cylindrical wall, the head (322, 422) having a first curved cut-out (310, 410), **characterized in that** the head further comprises a second curved cut-out (315, 412) wherein the first curved cut-out and the second curved cut-out are disposed on opposite sides of the head;
the first curved cut-out having a first depth (D3, D5) in a first direction (D) toward the center axis (201, 301, 401) and a first length through the head in a second direction (L) transverse relative to the first direction (D) and the center axis (201, 301, 401), wherein the first curved cut-out comprises a uniform profile such that a shape of the first curved cut-out is the same along the first length, and
the second curved cut-out having a second depth (D4, D6) in a third direction (D) toward the center axis (201, 301, 401) and a second length through the head in the second direction (L), wherein the second curved cut-out comprises a uniform profile such that a shape of the second curved cut-out is the same along the second length.

2. The needleless connector of Claim 1, wherein the first and second curved cut- outs have a common width (W).

3. The needleless connector of Claim 1, wherein a point of maximum depth of the second curved cut-out is axially offset along the center axis (201, 301, 401) from a point of maximum depth of the first curved cut-out.

4. The needleless connector of Claim 1, wherein the collapsible valve (200, 300, 400) comprises a diameter (D1), and the first depth is greater than or equal to thirty percent of the diameter, and the second depth (D4, D6) is less than or equal to twenty-five percent of the diameter.

5. The needleless connector of Claim 4, wherein the first depth is greater than or equal to fifty percent of the diameter, and the second depth is less than or equal to twenty percent of the diameter.

6. The needleless connector of Claim 1, wherein the first curved cut-out (310, 410) has a first width and the second curved cut-out (315, 412) has a second width that is less than the first width.

7. The needleless connector of Claim 1, wherein at least one profile of the first and second curved cut-outs (310, 410, 315, 412) each comprises a constant radius.

8. The needleless connector of Claim 1, wherein at least one profile of the first and second curved cut-outs (310, 410, 315, 412) comprises a variable radius.

9. The needleless connector of Claim 1, wherein at least one profile of the first and second curved cut-outs (310, 410, 315, 412) comprises a parabola.

10. The needleless connector of Claim 1, wherein the body (150) comprises a length of 3,048 cm (1.2 inches) or less.

11. The needleless connector of Claim 1, wherein the first length is measured at a maximum depth of the first curved cut-out.

12. The needleless connector of Claim 1, wherein the second length is measured at a maximum depth of the second curved cut-out.

## Patentansprüche

1. Nadelloser Verbinder, aufweisend:
einen Korpus (150), der einen inneren Hohlraum (151) aufweist, eine Öffnung (152), einen Ausgangsströmungskanal und einem Fluidströmungspfad zwischen der Öffnung (152) und dem Ausgangsströmungskanal; und
ein zusammendrückbares Ventil (200, 300, 400), das innerhalb des inneren Hohlraums (151) angeordnet ist, wobei das zusammendrückbare Ventil (200, 300, 400) aufweist:
eine Zylinderwand, die eine Mittelachse (201, 301, 401) und einer Schulter aufweist und einen inneren Luftraum bildet, wobei die Schulter dafür ausgelegt ist, einen Steg des Korpus (150) auf abdichtende Weise zu berühren, um den Fluidströmungspfad zu blockieren; und
einen Kopf (322, 422), der fest an der Zylinderwand angebracht ist, wobei der Kopf (322, 422) einen ersten gekrümmten Einschnitt (310, 410) aufweist, **dadurch gekennzeichnet, dass** der Kopf ferner einen zweiten gekrümmten Einschnitt (315, 412) aufweist, wobei er erste gekrümmte Einschnitt und der zweite gekrümmte Einschnitt auf gegenüberliegenden Seiten des Kopfes angeordnet sind;
wobei der erste gekrümmte Einschnitt eine erste Tiefe (D3, D5) in einer ersten Richtung (D) zur Mittelachse (201, 301, 401) hin und eine erste Länge durch den Kopf in einer zweiten Richtung (L) quer zur ersten Richtung (D) und zur Mittelachse (201, 301, 401) aufweist, wobei der erste gekrümmte Einschnitt ein gleichmäßiges Profil aufweist, so dass die Form des ersten gekrümmten Einschnitts entlang der ersten Länge gleich ist, und
wobei der zweite gekrümmte Einschnitt eine zweite Tiefe (D4, D6) in einer dritten Richtung (D) zur Mittelachse (201, 301, 401) hin und eine zweite Länge durch den Kopf in der zweiten Richtung (L) aufweist, wobei der zweite gekrümmte Einschnitt ein gleichmäßiges Profil aufweist, so dass die Form des zweiten gekrümmten Einschnitts entlang der zweiten Länge gleich ist.

2. Der nadellose Verbinder nach Anspruch 1, wobei der erste und der zweite gekrümmte Einschnitt die gleiche Breite (W) aufweisen.

3. Der nadellose Verbinder nach Anspruch 1, wobei ein Punkt maximaler Tiefe des zweiten gekrümmten Einschnitts axial entlang der Mittelachse (201, 301, 401) gegenüber einem Punkt maximaler Tiefe des ersten gekrümmten Einschnitts versetzt ist.

4. Der nadellose Verbinder nach Anspruch 1, wobei das zusammendrückbare Ventil (200, 300, 400) einen Durchmesser (D1) aufweist und die erste Tiefe größer oder gleich dreißig Prozent des Durchmessers ist und die zweite Tiefe (D4, D6) kleiner oder gleich fünfundzwanzig Prozent des Durchmessers ist.

5. Der nadellose Verbinder nach Anspruch 4, wobei die erste Tiefe größer oder gleich fünfzig Prozent des Durchmessers ist und die zweite Tiefe kleiner oder gleich zwanzig Prozent des Durchmessers ist.

6. Der nadellose Verbinder nach Anspruch 1, wobei der erste gekrümmte Einschnitt (310, 410) eine erste Breite aufweist und der zweite gekrümmte Einschnitt (315, 412) eine zweite Breite aufweist, die kleiner ist als die erste Breite.

7. Der nadellose Verbinder nach Anspruch 1, wobei mindestens ein Profil des ersten und zweiten gekrümmten Einschnitts (310, 410, 315, 412) jeweils einen konstanten Radius aufweist.

8. Der nadellose Verbinder nach Anspruch 1, wobei mindestens ein Profil des ersten und zweiten gekrümmten Einschnitts (310, 410, 315, 412) einen variablen Radius aufweist.

9. Der nadellose Verbinder nach Anspruch 1, wobei mindestens ein Profil des ersten und zweiten gekrümmten Einschnitts (310, 410, 315, 412) eine Parabel aufweist.

10. Der nadellose Verbinder nach Anspruch 1, wobei der Korpus (150) eine Länge von 3,048 cm (1,2 Zoll) oder weniger aufweist.

11. Der nadellose Verbinder nach Anspruch 1, wobei die erste Länge an der maximalen Tiefe des ersten gekrümmten Einschnitts gemessen ist.

12. Der nadellose Verbinder nach Anspruch 1, wobei die zweite Länge an der maximalen Tiefe des zweiten gekrümmten Einschnitts gemessen ist.

## Revendications

1. Connecteur sans aiguille comprenant :
un corps (150) comportant une cavité interne (151), un orifice (152), un canal d'écoulement de sortie et un chemin d'écoulement de fluide entre l'orifice (152) et le canal d'écoulement de sortie ; et
une valve pliable (200, 300, 400) disposée à l'intérieur de la cavité interne (151), la valve pliable (200, 300, 400) comprenant :
une paroi cylindrique présentant un axe central (201, 301, 401) et un épaulement et définissant un espace d'air interne, dans lequel l'épaulement est configuré pour entrer en contact de manière étanche avec une arête du corps (150) de manière à bloquer le chemin d'écoulement de fluide ; et
une tête (322, 422) attachée de manière fixe à la paroi cylindrique, la tête (322, 422) comportant une première découpe courbe (310, 410), **caractérisé en ce que** la tête comprend en outre une deuxième découpe courbe (315, 412), la première découpe courbe et la deuxième découpe courbe étant disposées sur des côtés opposés de la tête ;
la première découpe courbe ayant une première profondeur (D3, D5) dans une première direction (D) vers l'axe central (201, 301, 401) et une première longueur à travers la tête dans une deuxième direction (L) transversale par rapport à la première direction (D) et à l'axe central (201, 301, 401), dans lequel la première découpe courbe présente un profil uniforme de telle sorte que la forme de la première découpe courbe est la même sur toute la première longueur, et
la deuxième découpe courbe ayant une deuxième profondeur (D4, D6) dans une troisième direction (D) vers l'axe central (201, 301, 401) et une deuxième longueur à travers la tête dans la deuxième direction (L), dans laquelle la deuxième découpe courbe présente un profil uniforme de telle sorte que la forme de la deuxième découpe courbe est la même sur toute la deuxième longueur.

2. Le connecteur sans aiguille selon la revendication 1, dans lequel les première et deuxième découpes courbes ont la mème largeur (W).

3. Le connecteur sans aiguille selon la revendication 1, dans lequel un point de profondeur maximale de la deuxième découpe courbe est décalé axialement le long de l'axe central (201, 301, 401) par rapport à un point de profondeur maximale de la première découpe courbe.

4. Connecteur sans aiguille selon la revendication 1, dans lequel la valve pliable (200, 300, 400) présente un diamètre (D1), et la première profondeur est supérieure ou égale à trente pour cent du diamètre, et la deuxième profondeur (D4, D6) est inférieure ou égale à vingt-cinq pour cent du diamètre.

5. Le connecteur sans aiguille selon la revendication 4, dans lequel la première profondeur est supérieure ou égale à cinquante pour cent du diamètre, et la deuxième profondeur est inférieure ou égale à vingt pour cent du diamètre.

6. Le connecteur sans aiguille selon la revendication 1, dans lequel la première découpe courbe (310, 410) présente une première largeur et la deuxième découpe courbe (315, 412) présente une deuxième largeur qui est inférieure à la première largeur.

7. Le connecteur sans aiguille selon la revendication 1, dans lequel au moins un profil des première et deuxième découpes courbes (310, 410, 315, 412) comprend chacun un rayon constant.

8. Le connecteur sans aiguille selon la revendication 1, dans lequel au moins un profil des première et deuxième découpes courbes (310, 410, 315, 412) présente un rayon variable.

9. Le connecteur sans aiguille selon la revendication 1, dans lequel au moins un profil des première et de la deuxième découpe courbe (310, 410, 315, 412) comprend une parabole.

10. Le connecteur sans aiguille selon la revendication 1, dans lequel le corps (150) a une longueur de 3,048 cm (1,2 pouce) ou moins.

11. Le connecteur sans aiguille selon la revendication 1, dans lequel la première longueur est mesurée à la profondeur maximale de la première découpe courbe.

12. Connecteur sans aiguille selon la revendication 1, dans lequel la deuxième longueur est mesurée à la profondeur maximale de la deuxième découpe courbe.
